Europäisches Patentamt

European Patent Office

Office européen des brevets

(19)

(11) Numéro de publication: **0 223 665 B1**

(12) **FASCICULE DE BREVET EUROPEEN**

(45) Date de publication de fascicule du brevet:
**02.01.92**

(21) Numéro de dépôt: **86402314.8**

(22) Date de dépôt: **16.10.86**

Le dossier contient des informations techniques présentées postérieurement au dépôt de la demande et ne figurant pas dans le présent fascicule.

(51) Int. Cl.⁵: **A61K 39/015**, G01N 33/569, G01N 33/577

(54) **Antigènes obtenus à partir de la phase intraérythrocytaire de Plasmodium falciparum, leur purification, leur dosage ainsi que celui de leurs anticorps, et vaccins contre le paludisme les renfermant.**

(30) Priorité: **28.10.85 FR 8515986**

(43) Date de publication de la demande:
**27.05.87 Bulletin 87/22**

(45) Mention de la délivrance du brevet:
**02.01.92 Bulletin 92/01**

(84) Etats contractants désignés:
**AT BE CH DE ES FR GB GR IT LI NL SE**

(56) Documents cités:
**EP-A- 0 112 784**
**EP-A- 0 136 215**
**WO-A-85/00977**
**GB-A- 2 099 300**
**GB-A- 2 114 288**

(73) Titulaire: **INSTITUT NATIONAL DE LA SANTE ET DE LA RECHERCHE MEDICALE (INSERM) 101, rue de Tolbiac F-75654 Paris Cédex 13(FR)**

(72) Inventeur: **Vernes, Alain 20 Façade de l'Esplanade F-59800 Lille(FR)**
Inventeur: **Dubrometz, Jean-François 6 rue de la Coquerie F-59310 Nomain(FR)**
Inventeur: **Fortier, Bernard A 1 Place du Maréchal Leclerc F-59800 Lille(FR)**
Inventeur: **Delplace, Patrick 25 Square du Portugal F-59000 Lille(FR)**

(74) Mandataire: **Lemoine, Michel et al Cabinet Michel Lemoine et Bernasconi 13 Boulevard des Batignolles F-75008 Paris(FR)**

IMMUNOLOGICAL REVIEWS, vol. 61, 1982, pages 245-269, Munksgaard, Copenhagen, DK; L.H. PERRIN et al.: "Immunity to asexual erythrocytic stages of Plasmodium falciparum: role of defined antigens in the humoral response"

EXPERIENTA, vol. 40, no. 12, décembre 1984, pages 1343-1350, Birkhäuser Verlag, Bâle, CH; L. PERRIN et al.: "Malaria: immunity, vaccination and immunodiagnosis"

BULLETIN OF THE WORLD HEALTH ORGANIZATION, vol. 61, no. 1, 1983, pages 105-112; L. R. DA SILVA et al.: "Plasmodium falciparum polypeptides released during in vitro cultivation"

## Description

L'invention concerne la surveillance du paludisme, plus particulièrement du paludisme provoqué par Plasmodium falciparum qui est la forme la plus grave de cette maladie chez l'homme et la vaccination contre cette maladie.

Plus précisément, l'invention a pour objet deux protéines du milieu de culture de Plasmodium falciparum, leur précurseur dans le parasite, le dosage de ces protéines et de leurs anticorps dans le sérum, ainsi que des vaccins utilisant ces protéines en tant que principe actif.

Il est rappelé que le paludisme est une fièvre périodique très répandue dans le monde et ayant fréquemment une issue fatale, due à un protozoaire de type Plasmodium, notamment Plasmodium falciparum. Le protozoaire est transmis à l'homme par un moustique (anophèle).

Après une forte régression due à l'utilisation massive d'insecticides, d'une part et à des traitements chimiques préventifs et curatifs chez l'homme, d'autre part la maladie progresse de nouveau, en raison d'une part de la résistance des insectes aux insecticides les plus répandus et d'autre part de la résistance progressive de la maladie elle-même aux medicaments largement utilisés tels que quinine et nivaquine.

Compte tenu de ces constatations alarmantes, les recherches visant à lutter contre le paludisme s'orientent maintenant dans le sens d'un traitement préventif, par vaccination, notamment contre la forme la plus dangereuse de cette maladie, celle due à Plasmodium falciparum.

Il est rappelé que ce parasite présente un cycle de vie complexe. Lors de la piqûre, le moustique injecte dans la circulation sanguine des sporozoïtes qui représentent la forme infectieuse du parasite. Les sporozoïtes pénètrent ensuite dans le foie où ils se multiplient sans réponse immunitaire. Au bout d'une dizaine de jours ils sont libérés à partir du foie sous forme de mérozoïtes qui infectent certains globules rouges où ils se multiplient, ce qui provoque l'éclatement de ces globules rouges avec apparition d'un accès fébrile. Les mérozoïtes ainsi libérés gagnent d'autres érythrocytes, ce qui conduit à d'autres accès fébriles périodiques.

Par ailleurs, certains des parasites issus des érythrocytes conduisent à des formes sexuées qui infectent le moustique. Ces formes, après fécondation dans l'estomac de l'insecte, conduisent à la formation de sporozoïtes qui migrent dans les glandes salivaires de l'insecte le rendant ainsi infectant.

Compte tenu du cycle de vie de Plasmodium falciparum, on pourrait donc envisager de protéger les sujets exposés soit par un vaccin antisporozoïte, soit par un vaccin antimérozoïte.

La première de ces solutions semble toutefois devoir poser des problèmes importants dans la mesure où le vaccin devrait être totalement efficace, c'est-à-dire conduire à la destruction de tous les sporozoïtes susceptibles de pénétrer dans l'organisme, faute de quoi l'infection pourrait se développer en raison de la production de mérozoïtes et de leur multiplication, en l'absence d'anticorps spécifiques.

Le demandeur a par conséquent orienté ses recherches vers la production d'un vaccin de type antimérozoïte qui serait capable d'inhiber en particulier le stade de réinvasion des érythrocytes. Il s'est également intéressé à la mise au point de tests permettant d'une part d'étudier l'immunisation en particulier de sujets ayant été à plusieurs reprises en contact avec l'agent pathogène par dosage de leurs anticorps, et d'autre part de suivre l'évolution de la maladie, notamment au cours d'un traitement chimique.

Dans le but d'identifier et d'isoler des antigènes susceptibles d'être utilisés dans les applications invoquées ci-dessus, le demandeur s'est plus particulièrement intéressé aux produits libérés à partir de globules rouges parasités.

Plus précisément, dans le but d'identifier les exoantigènes de Plasmodium falciparum susceptibles d'être immunologiquement actifs, le demandeur a purifié les immunoglobulines provenant d'un plasma humain inhibant in vitro l'invasion des globules rouges par les mérozoïtes et à utilisé l'extrait purifié pour immunoprécipiter les produits libérés dans le milieu de culture par une culture synchrone de Plasmodium falciparum en présence de méthionine marquée au $^{35}$S, et ceci à différents stages du cycle schizogonique.

Afin de permettre un déroulement complet de ce cycle et une totale libération des produits synthétisés par le parasite, le demandeur a eu l'idée d'étudier les érythrocytes infectés et le milieu de culture, tant au cours du marquage par la méthionine $^{35}$S, que pendant une période prolongée en milieu non marqué, appelé "milieu froid" où il y avait arrêt de la synthèse radioactive. L'ensemble de ces deux étapes est connu sous le nom de "marquage"/"chasse" (en anglais "pulse/chase").

Cette façon de procéder est très différente de la technique proposée par Rodriguez Da Silva et coll. (référence 18) pour effectuer une étude du même type, qui avaient analysé les cellules et les surnageants à la fin de la période de marquage de 6 heures.

Les détails expérimentaux concernant cette étude sont donnés dans la partie expérimentale (I) du présent mémoire.

Les résultats obtenus montrent que 4 antigènes majeurs (140, 126, 108 et 70 kDa) sont reconnus par les IgGs immunes en fin de marquage, dans les hématies. Deux d'entre eux (126 et 108 kDa) disparaîssent au cours des 12 premières heures de "chasse". Dans les milieux de culture, un produit majeur de 50 kDa (accompagné par un composant moins intense de 47 kDa) apparaît à 12 heures pour diminuer ensuite. Ces deux antigènes ne sont pas observés dans les hématies parasitées.

L'analyse par électrophorèse bidimensionnelle montre que la bande 50 kDa est formée par une seule protéine de pI 5,5 alors que la bande 47 kDa est résolue en deux taches à 5,9 et 6,1 environ.

La littérature fait déjà état d'antigènes de Plasmodium de nature protéique et dont les poids moléculaires s'étagent dans différents intervalles.

Ainsi, la demande de brevet européen EP-A-0.136.215 décrit des protéines antigéniques retrouvées dans différents types d'antigènes dits de surnageant, de lavage et de lavage hypotonique. Les poids moléculaires de ces antigènes s'étagent entre 35.000 et 80.000 daltons et deux immunogènes considérés comme principaux possèdent des poids de 42.000 respectivement 54.000, une bande de 49000 daltons se résolvant en 54.000 et 46.000 daltons. On notera tout d'abord que la simple indication d'un poids moléculaire ne permet pas de distinguer des antigènes et il est habituel d'observer, dans une fourchette de 1000 daltons autour d'un poids moléculaire donné, ce qui correspondant à la limite extrême de précision de l'analyse SDS PAGE, plusieurs protéines distinctes de plasmodium n'ayant aucune spécificité ou fonctionnalité antigénique commune entre elles. Si on essaye de mieux définir les antigènes décrits dans ce document antérieur, on relève que ces derniers sont de nature glycoprotéique et que les anticorps synthétisés par les animaux expérimentalement immunisés vis-à-vis de ces protéines, montrent que toutes les formes du parasite sont marquées, y compris les formes jeunes (rings). Par ailleurs, les sérums humains provoquent in vitro une inhibition de l'invasion des hématies saines par les mérozoïtes, ce qui indique que les antigènes cibles se trouvent à la surface des mérozoïtes.

Comme le montre la description faite ci-après de l'invention, les antigènes apparaissant dans l'invention ne correspondent à aucune de ces caractéristiques.

L'article EXPERIENTIA, vol. 40, no 12 décembre 1984, pages 1343-1350, Basel CH ; L. PERRIN et al. : "Malaria : immunity, vaccination and immunio-diagnosis" énumère des antigènes annoncés comme spécifiques par rapport aux schizontes et/ou mérozoïtes ayant différents poids moléculaires s'étageant de 35.000 à 200.000 daltons. Ces protéines n'ont pas de rapport avec celles apparaissant dans la présente invention.

La demande de brevet européen EP-A-0.112.784 concerne des protéines extraites du parasite après lyse des hématies. L'une des catégories est caractérisée par un poids moyen de l'ordre de 50.000 plus ou moins 5000 daltons et est extraite du parasite et non pas du milieu comme dans l'invention. Cette catégorie ne peut pas être mise en évidence par l'incorporation métabolique de méthionine 35-S alors que la protéine selon l'invention d'un poids moléculaire 50.000 daltons possède des propriétés exactement contraires comme cela apparaîtra dans la description suivante.

La demande de brevet britannique GB-A-2.099.300 a trait à des protéines de poids moléculaire allant de 180.000 à 250.000 daltons et associées aux membranes des schizontes érythrocytaires, métabolisées à l'intérieur des érythrocytes en fragments plus petits et associées à la membrane du mérozoïte. Ces protéines sont obtenues par solubilisation des érythrocytes infectés et il n'y a aucun rapport entre ces protéines et celles apparaissant dans la présente invention.

L'article de RODRIGUEZ DA SILVA et COLL (référence 18) précité a trait à des molécules provenant d'extraits du parasite et du milieu de culture. Les molécules identifiées dans le surnageant ont été produites après au plus 6 heures après marquage, c'est-à-dire bien avant l'apparition des antigènes apparaissant dans l'invention. Des anticorps monoclonaux spécifiques de ces molécules reconnaissent des protéines de poids moléculaires différents de ceux de l'invention et qui sont associées pour certaines d'entre elles, à tous les stades du parasites, y compris les stades jeunes de sorte que ces molécules sont totalement différentes de celles apparaissant dans l'invention.

La demande de brevet britannique GB-A-2.114.288 décrit une méthode de détection des antigènes circulants de Plasmodium par inhibition compétitive, les antigènes étant extraits des érythrocytes parasités et correspondant principalement aux formes jeunes. Ces antigènes présentent des réactions croisées avec les Plasmodium de rongeurs et les autres espèces de Plasmodium humain, ce qui n'est nullement le cas dans la présente invention.

La demande de brevet PCT WO-A-8.500.977 décrit des antigènes protéiques de 41.000 à 58.000 daltons avec des Pi de 4,7 à 5,5, ces protéines ne correspondant pas à celles apparaissant dans l'invention.

L'invention a donc pour objet des antigènes pouvant être isolés à partir de la phase intraérythrocytaire de Plasmodium falciparum, caractérisés en ce qu'ils consistent en :

a) une protéine de 126 kDa synthétisée pendant la phase de multiplication nucléaire au cours de la schizogonie et localisée à la périphérie des schizontes, au niveau de la vacuole parasitophore ;

b) une protéine de 50 kDa présentant un point isoélectrique de 5,5 apparaissant dans le sérum des malades infectés par Plasmodium falciparum ou dans le milieu de culture de microorganisme, au cours de sa phase intraérythrocytaire et ayant pour précurseur la protéine de 126 kDa définie sous a) ; et

c) une protéine de 47 kDa se résolvant, par électrophorèse, en deux taches à 5,9 et 6,1 environ, apparaissant dans le sérum des malades infectés par Plasmodium falciparum ou dans le milieu de culture de ce microorganisme, au cours de sa phase intraérythrocytaire et ayant pour précurseur la protéine de 126 kDa définie sous a).

On a également constaté que la protéine de 126 kDa est le précurseur d'une protéine de 18 kDa apparaissant dans le sérum des malades infectés par Plasmodium falciparum ou dans le milieu de culture de ce micro-organisme au cours de sa phase intra-érythrocytaire.

Par ailleurs, à l'état natif, la protéine de 47 kDa et la protéine 18 de kDa précitées sont réunies en une protéine d'environ 65 kDa par des ponts disulfures et l'invention a donc également pour objet un antigène consistant en une telle protéine.

Les poids moléculaires de 126 kDa et 47 kDa définis ci-dessus ont été déterminés sur une souche particulière de Plasmodium falciparum, à savoir la souche FCR-3 (référence 1) et l'on constate que lorsque l'on utilise d'autres souches, ces poids moléculaires peuvent légèrement varier, généralement entre 126 et 130 kDa, respectivement 47 et 50 kDa. Ainsi, par exemple, on constate des valeurs de 130 kDa et 50 kDa, cette dernière se résolvant par électophorèse en deux taches à 5,9 et 6,1 environ, lors de l'isolation à partir de la souche de Plasmodium falciparum ITUX-1 publiquement disponible.

En conséquence, dans la suite de la description ainsi que dans les revendications, la définition a) "protéine 126 kDa" signifie toute protéine correspondante et dont le poids moléculaire, en fonction des souches, est compris, notamment entre 126 kDa à 130 kDa, et c) "protéine 47 kDa" signifie également toute protéine correspondante en fonction des souches et d'un poids moléculaire compris notamment entre 47 kDa et 50 kDa.

D'autre part, par antigène selon l'invention, on entend également les antigènes peptidiques immunogènes dérivant des différents antigènes précités et reconnus par un sérum polyclonal spécifique de la protéine 126 kDa précitée.

On peut, selon des techniques classiques préparer, à partir du surnageant d'une culture parasitaire de Plasmodium falciparum, un antigène obtenu par chromatographie d'affinité sur une colonne portant des IgGs de sérum hyper-immun et utiliser cet antigène pour obtenir des hybridomes murins produisant des anticorps spécifiques soit de la molécule 50 kDa, soit de la molécule 47 kDa décrites ci-dessus.

Ces anticorps, qu'ils soient spécifiques de la molécule 50 kDa ou de la molécule 47 kDa, reconnaissent également leur précurseur commun dans le parasite, à savoir la molécule 126 kDa.

Un exemple d'obtention et de sélection de tels hybridomes est donné à titre indicatif dans la partie expérimentale (II) du présent mémoire.

Les molécules 50 kDa, 65 kDa, 47 kDa et 18 kDa peuvent être purifiées en faisant passer le surnageant du milieu de culture asynchrone de Plasmodium falciparum sur une colonne de chromatographie d'affinité, notamment de Sépharose 4B (Pharmacia), couplée avec des anticorps spécifiques de la molécule 50,65,47 ou 18 kDa respectivement et obtenus de façon classique à partir des hybridomes produisant les anticorps monoclonaux correspondants. A titre indicatif, les anticorps peuvent être présents sur la colonne à raison d'environ 10 mg d'IgG par ml de gel.

De même, la molécule 126 kDa peut être purifiée par solubilisation d'hématies parasitées par Plasmodium falciparum, par exemple au moyen de déoxycholate de sodium à 0,5% et en faisant passer la solution obtenue sur une colonne munie d'anticorps monoclonaux reconnaissant spécifiquement la molécule 50 kDa et/ou la molécule 47 kDa et/ou 18 kDa et/ou 65 kDa.

Des anticorps polyclonaux spécifiques sont aussi utilisables pour ces purifications.

Des exemples de telles purifications sont donnés dans la partie expérimentale (III).

Selon un autre de ses aspects, l'invention fournit un procédé pour le dosage des anticorps contre les protéines selon l'invention, notamment les molécules 50 65, 18 et 47 kDa, dans le sérum de sujets immunisés ou en cours d'immunisation.

Différentes techniques bien connues de dosage faisant intervenir des réactions immunologiques peuvent être utilisées, comme par exemple la technique RIA (Radio Immuno Assay) utilisant un marqueur radioactif.

On préfère toutefois, pour des raisons de simplicité et de sécurité du personnel, avoir recours à une technique mettant en oeuvre, en tant que marqueur une ou plusieurs enzymes. On citera par exemple dans ce contexte les différentes méthodes ELISA (Enzym Linked Immuno Sorbent Assay) connues.

Une technique de ce type qui donne de bons résultats selon l'invention comprend essentiellement les étapes consistant à :

1) fixer sur un support un anticorps monoclonal spécifique de la molécule (18, 47, 65, 50 et/ou 126 kDa) correspondant à l'anticorps à doser ;

2) saturer les sites libres restant sur le support ;

3) faire réagir avec un excès d'antigène obtenu à partir du surnageant d'une culture asynchrone de Plasmodium falciparum ;

4) faire réagir l'antigène fixé sur le support à l'étape 3) avec le milieu à tester ;

5) faire réagir l'ensemble avec un anticorps anti-immunoglobulines humaines, marqué par une enzyme ;

6) déterminer la quantité d'anticorps spécifiques présente dans le milieu étudié, par l'intermédiaire de la quantité d'enzyme fixée sur le support à l'étape 5), au moyen d'une réaction spécifique de l'enzyme, grâce à un étalonnage préalable.

L'invention fournit également un procédé de dosage des molécules 18,47,65 et 50 kDa dans le sérum des malades, notamment lors d'un accès de paludisme, ou dans le milieu de culture, ainsi qu'un procédé de dosage de la molécule 126 kDa dans les érythrocytes, après solubilisation.

Dans tous les cas, on a recours à des dosages immunologiques, notamment de type ELISA.

Selon un mode préféré de réalisation, un tel dosage comprend essentiellement les étapes consistant à :

1) fixer sur un support un anticorps monoclonal spécifique de la molécule à doser ;

2) saturer les sites libres restant sur le support ;

3) faire réagir avec le milieu susceptible de contenir l'antigène à doser ;

4) faire réagir l'ensemble avec du sérum de référence provenant d'un sujet immun ;

5) faire réagir avec une antiglobuline anti IgG humaines, marquée par une enzyme ;

6) déterminer la quantité d'antigène présente dans le milieu étudié, par l'intermédiaire de l'activité de l'enzyme, au moyen d'un étalonnage préalable.

Dans la partie expérimentale (IV à VI) sont décrits, à titre purement illustratif, des dosages de ce type concernant les molécules 50 kDa et 47 kDa.

L'évolution du paludisme chez un malade peut être étudiée par dosage dans son sérum, à intervalles réguliers, des antigènes selon l'invention, par exemple comme indiqué dans la partie expérimentale (VII).

Les travaux du demandeur ont conduit à définir l'unité de protéine 50 kDa (U 50) comme étant la quantité de molécule 50 kDa libérée en 24 heures par une culture de la souche FCR-3 de Plasmodium falciparum, dans un volume de 1 ml, à l'hématocrite de 6% et à une parasitémie initiale de 7 %.

Selon un autre de ses aspects, l'invention fournit un vaccin contre le paludisme, caractérisé en ce qu'il comporte, en tant que principe actif, au moins l'une des protéines 18, 47, 50,65 et 126 kDa, de préférence en présence d'un adjuvant.

De préférence, on utilise chacun des antigènes seul ou alternativement les antigènes 50 kDa et 47 kDa ensemble, par exemple en des quantités égales.

L'adjuvant est de préférence l'hydroxyde d'aluminium (Al(OH)$_3$).

Selon un mode préféré de réalisation de l'invention, une dose de vaccin de 0,5 ml contient :

- de 0,5 à 1,5 mg, de préférence 1 mg, de protéine 50 kDa et/ou 47 kDa, ou 126 kDa ou encore 18 et/ou 65 kDa.
- de 0,3 à 2 mg, de préférence 1 mg, d'hydroxyde d'aluminium et
- une quantité suffisante de soluté isotonique tamponné.

A titre d'exemple, un protocole de vaccination avantageux consiste à effectuer trois injections successives d'une dose de vaccin, à des intervalles de 1 mois, avec un rappel un an après la troisième injection.

Avantageusement, l'injection est effectuée par voie sous-cutanée dans la fosse sous-épineuse ou encore dans la face externe de l'avant-bras ou de la cuisse.

Une injection intra-musculaire peut également être envisagée.

Plus de détails concernant l'invention et des exemples d'application, donnés à titre purement illustratifs, sont donnés dans la partie expérimentale qui suit.

I - ANALYSE DES ANTIGENES LIBERES DANS LE MILIEU DE CULTURE DE PLASMODIUM FALCIPARUM AU COURS D'UN CYCLE ERYTHROCYTAIRE

1. Culture du parasite et synchronisation :

On cultive la souche FCR-3 de Plasmodium falciparum (référence 1) dans des globules rouges humains O$^+$ à 6% d'hématocrite, 10 % de parasitémie, dans du milieu RPMI 1640 (référence 2) additionné de 10 % de sérum humain A$^+$ (référence 3). La culture est synchronisée par deux traitements au sorbitol espacés de 27 heures (référence 4).

2. Inhibition de la réinvasion

On procède selon la méthode de A. Vernes et coll. (référence 4). Des cultures synchronisées contenant des schizontes (100 $\mu$l par essai, 1,5% d'hématocrite, 1% de parasitémie) sont exposées à des IgGs purifiées normales ou immunes à différentes concentrations pendant 18 h, on lave et on cultive dans du milieu normal pendant encore 8h, puis pendant 16 h dans un milieu additionné de précurseur radioactif (isoleucine $^{14}$C ou hypoxanthine $^3$H). On recueille alors les globules rouges parasités avec de l'eau distillée et on récupère la matière insoluble sur des fibres de verre (récupérateur de cellules de la société Mash). On mesure la radioactivité dans un liquide organique (mélange de solvants) du type Aqualuma de la société Lumac, au moyen d'un compteur à scintillation de type LS-1800 Beckman.

## 3. Marquage par la méthionine $^{35}$S

Les érythrocytes obtenus en 1 sont remis en suspension dans du milieu de culture à un hématocrite de 1 % et distribués, sous des volumes de 5 ml, dans des fioles de culture de 25 cm$^3$. 30 heures après le second traitement au sorbitol, les érythrocytes sont lavés dans du milieu ne contenant pas de méthionine (RPMI 1640, "Selactamine kit" de la Société GIBCO) et incubés pendant 5 heures dans le même milieu additionné de 40 $\mu$Ci ml$^{-1}$ de méthionine $^{35}$S (>800 Ci mmol$^{-1}$, Amersham). Une fiole contenant des globules rouges non parasités est traitée de façon identique en tant que témoin. A la fin de la période de marquage (t=0), l'une des fioles est centrifugée (400 g, 10 minutes), le surnageant est recueilli et les cellules parasitées marquées sont remises en suspension dans 5 ml de milieu non radioactif dit "milieu froid" et réincubées à 37°C. Le surnageant est ensuite remplacé dans les mêmes conditions toutes les 12 heures pendant 48 heures. Un second flacon est traité de manière identique mais, à chaque étape, une partie des hématies parasitées est également collectée : on obtient ainsi cinq paires d'échantillons au bout de 0, 12, 24, 36 et 48 heures respectivement après la fin de la période de marquage.

Tous les surnageants de culture sont centrifugés à 20000 g pendant 1 heure immédiatement après la récolte. Les cellules sont lavées et remises en suspension dans du milieu RPMI sans sérum. Les surnageants et les suspensions de cellules sont ajustés à 0,6 % de dodécylsulfate de sodium (SDS) et chauffés à 100°C pendant 10 minutes. Après refroidissement, on ajuste à 0,5 % de SDS et 2,5 % de Triton X-100 (dénomination commerciale pour un agent tensio-actif de la Société ROHM & HAAS), selon la méthode de Erickson et Blobel (référence 5).

## 4. Purification de l'IgG et préparation de l'immunosorbant

Les immunoglobulines ont été purifiées à partir du plasma d'un donneur européen vivant dans une zone de malaria endémique (Madagascar) et à partir du plasma d'un témoin non immun, par chromatographie d'échange d'ions sur du DEAE trisacryl (Industrie Biologique Française) selon la méthode de Saint-Blancard et coll. (référence 6). La concentration des IgG est mesurée par néphélométrie au laser (référence 7).

Les immunosorbants sont préparés par couplage de ces IgGs sur de la Sépharose 4B (Pharmacia), activée au bromure de cyanogène à raison de 6 mg de protéines par ml de gel, selon les recommandations du fabricant.

## 5. Immunosorption

Chaque échantillon solubilisé (correspondant soit à 10 $\mu$l de cellules parasitées soit à 5 ml de milieu) est appliqué (en utilisant un système de recyclage) à deux colonnes successives contenant 100 $\mu$l d'IgGs couplées à la Sépharose 4B, la première étant préparée avec des IgGs non immunes (pour l'adsorption non spécifique), la seconde avec des IgGs inhibitrices (pour l'immunosorption). Chaque gel est ensuite lavé cinq fois avec du tampon contenant 0,5% de SDS et 2,5 % de Triton X-100, et élué à 100°C pendant 3 minutes dans du tampon pour échantillon pour électrophorèse sur gel de polyacrylamide-SDS (SDS-PAGE) (référence 8).

## 6. SDS-PAGE et fluorographie

L'analyse SDS-PAGE à une dimension est effectuée dans des gels d'acrylamide à 12 % selon la méthode de Laemmli (référence 8). La fluorographie est effectuée selon la méthode de R.A. Laskey et coll. (référence 9).

Les résultats obtenus montrent que 4 antigènes majeurs (140, 126, 108 et 70 kDa (kiloDaltons)) sont reconnus par les IgGs immunes en fin de marquage, dans les hématies. Deux d'entre eux (126 et 108 kDa) disparaissent au cours des 12 premières heures de traitement en "milieu froid", traitement appelé "chasse". Dans les milieux de culture, un produit majeur de 50 kDa (accompagné par un composant moins intense de 47 kDa) apparaît à 12 heures pour diminuer ensuite. Ces deux antigènes ne sont pas observés dans les hématies parasitées.

7. Electrophorèse bidimensionnelle

L'éluat de l'immunosorbant contenant le surnageant obtenu au bout de 12 heures de "chasse" est complété par 0,3 % de SDS, de l'urée 9,95 M, 4 % de Nonidet P-40 (détergent de la Société Shell), 2 % d'Ampholines (de la Société LKB, pH 3,5-10) et du dithiothréitol 0,1 M (référence 10). On l'analyse ensuite par la technique de PAGE bidimensionnelle selon la méthode de O'Farrel (référence 11).

Cette analyse par électrophorèse bidimensionnelle montre que la bande de 50 kDa est formée par une seule protéine de point isoélectrique 5,5 alors que la bande de 47 kDa est résolue en deux taches (spots) à 5,9 et 6,1 environ.

La radioactivité mesurée après précipitation par l'acide trichloroacétique (TCA) obtenue à partir du surnageant et d'hématies parasitées récoltées pendant 48 heures après un marquage de 5 heures par la méthionine $^{35}$S et à partir des éluats immunoadsorbés correspondants, est montrée dans le tableau I qui suit.

La distribution des stades parasitaires est également reportée pour chaque étape de prélèvement.

TABLEAU I

| Radioactivité[a] (c.p.m.) | Temps de "chasse" (heures) | | | | |
|---|---|---|---|---|---|
| | 0 | 12 | 24 | 36 | 48 |
| Total (x10$^{-6}$) | | | | | |
| Milieu de culture | 2,22 | 11,2 | 7,2 | 2 | 1,1 |
| Erythrocytes | 63,6 | 20,8 | 9,2 | 8,2 | 2,2 |
| Eluats immunoadsorbés[b] (x10$^{-3}$) | | | | | |
| Milieu de culture | 147(1) | 882(1,5) | 167(1,6) | 38,5(0,8) | 18,9(0,6) |
| Erythrocytes | 3944(62) | 564(36) | 302(20,4) | 282(14) | 43(25) |
| Stades parasitaires[c] | | | | | |
| Anneaux | 3,5 | 89 | 55 | 26 | 46 |
| Trophozoïtes | 81 | 4 | 43 | 72 | 43 |
| Schizontes | 15,5 | 7 | 2 | 2 | 11 |

a. Chaque valeur reportée correspond au contenu total du flacon de culture
b. Valeurs d'adsorption non spécifiques données entre parenthèses
c. Pourcentage de parasitémie totale

II - OBTENTION D'HYBRIDOMES MURINS SPECIFIQUES DES DEUX MOLECULES 50 kDa ET 47 kDa EXCRETEES PAR PLASMODIUM FALCIPARUM EN CULTURE IN-VITRO ET DE LEUR PRECURSEUR COMMUN DANS LE PARASITE

9

1. Matériel et méthodes

a) Les souris.

Tant pour l'obtention de lymphocytes immuns que pour la production in vivo des anticorps monoclonaux, des souris BALB/C (IFFA-CREDO) ont été utilisées. Elles étaient âgées de 6 à 12 semaines.

b) L'antigène.

L'antigène servant à immuniser les souris a été obtenu par culture de la souche FCR-3 de Plasmodium falciparum sur hématies humaines de groupe O Rh positif en milieu RPMI 1640 supplémenté avec 10 % de sérum humain de groupe A selon la technique de Trager et Jensen (référence 3). La culture parasitaire a été synchronisée par deux traitements au sorbitol selon Vernes et coll. (référence 4). L'antigène a été purifié à partir de 50 ml de surnageant recueilli juste après la réinvasion alors que l'hématocrite de la culture était de 2 % et que 10 % des hématies contenaient des schizontes. Une purification par affinité à été réalisée en passant ce surnageant sur une colonne préparée avec les IgGs d'un sérum humain hyperimmun servant de référence.

Le matériel obtenu, après avoir été réparti en trois fractions aliquotes égales, a servi à immuniser une souris selon le protocole suivant : deux injections intrapéritonéales avec de l'adjuvant complet de Freund aux jours 0 et 21, suivies d'une injection intraveineuse 7 jours plus tard.

c) Fusion cellulaire et culture des hybrides.

Trois jours après la troisième injection, la souris a été sacrifiée et la rate prélevée stérilement. Après broyage de la rate et lavage des cellules ainsi obtenues en milieu sans sérum, les splénocytes ont été numérés et mélangés à des cellules de myélome murin non sécrétant NS-1 (référence 12), dans un rapport de 10 splénocytes pour une cellule NS-1. La fusion a été favorisée par l'utilisation de polyéthylèneglycol 1500 (Merck) et de diméthylsulfoxyde et réalisée selon la technique décrite par Galfre et coll. (référence 13). Après la fusion, les cellules, lavées en présence de sérum de veau foetal (SVF) ont été réparties uniformément dans les cupules de 4 plaques de microtitration à fond plat (Costar, réf. 12 905-96, Flobio, France) contenant depuis 24 heures une couche nourricière de macrophages péritonéaux de souris BALB/C non-immunes (5000 cellules par cupule) (référence 14).

Pendant 24 heures la culture s'est déroulée en milieu de Eagle modifié selon Dulbecco (référence 15) et contenant 10 % de SVF. Ce milieu a été supplémenté avec des acides aminés non essentiels, du pyruvate de sodium, de la l-glutamine, de la pénicilline, de la streptomycine ainsi que de l'hypoxanthine et de la thymidine (milieu appelé ci-après HT).

A partir du lendemain les cellules myélomateuses non fusionées ont été éliminées en ajoutant de l'aminoptérine au milieu HT pour obtenir un milieu HAT selon Littlefield (référence 16).

Les cellules des puits de fusion positifs lors des épreuves de sélection ont été clonées deux fois par dilution limite. Après le premier clonage, les cellules retenues ont été progressivement sevrées en aminoptérine et cultivées en milieu HT.

Les cellules ont été congelées selon le protocole suivant : réfrigération à +4°C, centrifugation à 400 g pendant 10 minutes, reprise du culot de centrifugation dans un mélange DMSO 10 % et SVF 90 % préalablement maintenu à 0°C, répartition en fractions aliquotes. Les tubes (Nunc, Bio-Block, France) ont été déposés à -80°C pendant 24 heures puis stockés dans l'azote liquide.

d) Sélection des hybrides.

Plusieurs tests ont été systématiquement utilisés tout au long de ce travail pour sélectionner les hybrides.

Immunofluorescence (IF) et immunoélectrotransfert ("blot") avec des surnageants de cultures de P. falciparum, souche FCR-3. Les hybridomes ont été systématiquement caractérisés par immuno-précipitation d'une part d'extraits solubilisés par un détergent (Nonidet P-40) de parasites marqués pendant 5 heures par de la méthionine $^{35}$S et d'autre part du surnageant d'une culture marquée pendant 5 heures par de la méthionine $^{35}$S et "chassée" pendant 12 heures.

Un sérum de lapin anti-IgGs murines couplé à de la protéine A-Sépharose 4B (Pharmacia) a été utilisé. L'électrophorèse en gel de polyacrylamide (SDS-PAGE) a été révélée par fluorographie (immunoprécipitation).

e) Production des IgGs monoclonales purifiées.

Pour la production en masse des anticorps monoclonaux, les souris ont été d'abord inoculées par voie intrapéritonéale avec 0,5 ml de pristane (2, 6, 10, 14-tétraméthylpentadécane) (Serva, Tebu, France). 15 jours plus tard, les cellules ($5.10^6$) étaient injectées par la même voie.

Les ascites ont été recueillies à partir du 15e jour, regroupées et centrifugées à 1500 g pendant 30 minutes à +4°C.

Les IgGs ont été purifiées par chromatographie échangeuse d'ions en colonne DEAE-TRISACRYL (IBF France). Après contrôle de la pureté par électrophorèse en gel de polyacrylamide, les concentrations protéiques ont été mesurées au moyen du "Bio Rad Protein Assay" de la Société BIO-RAD. Les échantillons de ces IgGs ont été conservés à -80 °C jusqu'à leur utilisation.

2. Résultats

Parmi les 39 hybridomes ayant présenté une activité immunologique spécifique de Plasmodium falciparum, 2 ont été plus spécialement retenus en raison de leur spécificité pour les molécules 50 kDa et 47 kDa excrétées par Plasmodium falciparum en culture et leur précurseur commun dans le parasite.

Les caractéristiques de ces deux hybridomes sont résumées dans le tableau II suivant :

## TABLEAU II

CARACTERISATION DES HYBRIDOMES SPECIFIQUES DES MOLECULES 50 kDa ET 47 kDa EXCRETEES DANS LE MILIEU DE CULTURE PAR PLASMODIUM FALCIPARUM ET DE LEUR PRECURSEUR INTRACELLULAIRE

| Hybridome | 23D5 2H6 | 3E9 1A11 |
|---|---|---|
| Sous-classe | IgG$^*$ | IgG$_1$ |
| Molécule reconnue dans les parasites ("Blot" et immuno-précipitation) | 126 kDa | 126 kDa |
| Molécule reconnue dans le milieu de culture ("Blot" et immunoprécipitation) | 50 kDa (126 kDa$_{**}$ traces ) | 47 kDa (126 kDa$_{**}$ traces ) |
| Immunofluorescence sur étalement d'hématies parasitées | Périphérie des schizontes âgés et des mérozoïtes intra cellulaires | |

$^*$ vraisemblablement IgG$_2$
$^{**}$ Dans les conditions de culture, la transformation 126-50 kDa n'est pas totale, ce qui peut refléter une inadéquation partielle des conditions de culture in vitro à la multiplication optimale des parasites.

### III- PURIFICATION DES ANTIGENES 50 kDa, 47 kDa et 126 kDa

1. Purification de l'antigène 50 kDa

Le milieu de culture est d'abord passé sur une colonne de Sépharose 4B (Pharmacia) ne portant pas de protéine, puis sur une colonne de Sépharose 4B couplée à des anticorps monoclonaux produits par l'hybridome 23D5 2H6 du tableau II, à raison de 10 mg d'IgG par ml de gel.

Après lavage, on élue par la diéthylamine 0,1 M, pH 11,2, effectue une dialyse et concentre.

2. Purification de l'antigène 47kDa

On procède de la même manière en utilisant une colonne munie d'anticorps monoclonaux produits par l'hybridome 3E9 1A11 du tableau II.

11

3. Purification de l'antigène 126 kDa

On procède comme précédemment en remplaçant le milieu de culture par des hématies parasitées solubilisées dans du déoxycholate de sodium à 0,5 % et en utilisant l'un ou l'autre des anticorps monoclonaux indiqués plus haut.

IV - DOSAGE DES ANTICORPS SPECIFIQUES DE LA MOLECULE 50 kDa EXCRETEE PAR PLASMODIUM FALCIPARUM DANS LE MILIEU DE CULTURE IN-VITRO PAR UNE TECHNIQUE IMMUNO-ENZYMATIQUE (ELISA)

1. Matériel

a) L'anticorps monoclonal.
Le clone 23D5 2H6 a été retenu pour le dosage des anticorps humains anti 50 kDa de Plasmodium falciparum.
b) L'antigène.
L'antigène servant au dosage des IgGs humaines spécifiques par immunocapture est un surnageant de culture asynchrone de Plasmodium falciparum. Les conditions de culture sont les suivantes :
Temps 0 :
hématocrite 6 % (hématies humaines O Rh + )
parasitémie 7 % (souche FCR-3)
milieu neuf
Récolte à la 24e heure
La culture est alors centrifugée (600 g pendant 30 minutes à + 4°C). Le surnageant ainsi recueilli est réparti en fractions aliquotes et congelé à -80°C jusqu'au moment de l'utilisation.
c) Le sérum de référence.
De manière à diminuer les variations des dosages au cours de manipulations différentes, le résultat de chaque sérum a été comparé à celui d'un sérum de référence testé systématiquement en parallèle sur chaque plaque.
Ce sérum de référence est le sérum hyper-immun préalablement décrit, il a été considéré ici comme étant à "100 % d'anticorps".
Un sérum d'enfant âgé de 7 ans ayant toujours vécu en France métropolitaine, sans aucun antécédent pathologique connu, a également été testé sur chaque plaque. Il servait à mesurer le bruit de fond.
d) Les sérums testés.
178 sérums ont été testés au cours de cette étude. Ils provenaient de patients d'âge connu et sont répertoriés dans le tableau III.
50 provenaient de France et avaient été prélevés soit sur des nouveaux-nés (5) soit sur des adultes jeunes (45) n'ayant aucun antécédent connu de paludisme (groupe témoin).
108 sérums avaient été prélevés au Gabon dans la région de Franceville. La répartition des âges est donnée dans le tableau III.
20 sérums d'origines géographiques diverses provenaient de patients souffrant d'une primo-invasion palustre.
Ces sérums ont été répartis en échantillons dès leur prélèvement et maintenus à -20°C jusqu'à leur utilisation.
Pour 38 sérums, pris dans ces trois catégories, il a été possible de réaliser une immuno-précipitation tant à partir du milieu de culture qu'à partir des hématies parasitées par Plasmodium falciparum souche FCR-3. La technique d'immuno-précipitation est décrite plus haut.
e) La technique ELISA utilisée.
La quantification des anticorps anti 50 kDa de Plasmodium falciparum a été réalisée en plaques de microtitration à fond plat (Costar, Flobio, France).
Ces plaques ont été sensibilisées sous un volume de 200 $\mu$l, sans prétraitement préalable, par une solution de 6,25 gamma/ml d'IgGs purifiées obtenues à partir de l'hybridome 23D5 2H6 en tampon PBS (tampon phosphate salin) 0,15M, pH 9,6. Après une incubation à + 4°C, supérieure à 18 heures, les cupules ont été lavées avec de l'eau déminéralisée contenant 0,05 % de Tween 20 (dénomination commerciale pour du laurate de sorbimacrogol, Serva, Tebu, France), puis séchées rapidement par retournement (lavage). Elles ont été saturées pendant 2 heures à 37°C par une solution à 5 % d'albumine bovine en tampon PBS pH 7,2 0,15 M (PBS) (200 $\mu$l par cupule).

12

Après un nouveau temps de lavage, l'immunocapture de l'antigène a été réalisée en diluant ce dernier à 1/10 dans du PBS contenant 0,1 % de Tween 20 et 1 % d'albumine bovine (PBS-T-A). 200 $\mu$l de cette dilution ont été répartis dans chaque cupule, les plaques étant alors laissées à +4°C pendant 18 heures.

Après un nouveau lavage les sérums, dilués à 1/200 en PBS-T-A (PBS-Tween-Albumine), ont été déposés sous un volume de 200 $\mu$l par cupule en duplicat et laissés incuber 2 heures à 37°C.

Un lavage identique a précédé le dépôt de l'antiglobuline conjuguée à la peroxydase (anticorps de chèvre anti chaîne gamma humaine), (Tago, Biosoft, Paris): 200 $\mu$l par cupule, dilution 1/3000 en PBS-T-A. Après une incubation de 90 minutes à 37°C, les plaques ont été lavées et la présence de la peroxydase révélée par oxydation de l'ortho-phénylène diamine (OPD) en versant dans chaque cupule 200 $\mu$l de la solution suivante :

tampon citrate-phosphate pH 5 ........ 10 ml

ortho-phénylène diamine .............. 4mg

eau oxygénée 110V.................... 15 $\mu$l

(préparation extemporanée).

Après une incubation de 10 minutes à température ambiante et à l'obscurité, la réaction enzymatique a été arrêtée par un apport de 50 $\mu$l d'acide sulfurique 2N dans chaque cupule. La densité optique de chaque cupule a été mesurée directement à travers la plaque par un photomètre (DYNALAB) à la longueur d'onde de 492 nm.

f) Expression des résultats.

Pour chaque sérum étudié, la densité optique retenue (DO) a été la moyenne de celles des deux puits correspondants, diminuée du bruit de fond mesuré dans les mêmes conditions à partir du sérum négatif. Cette DO a été comparée à celle du sérum de référence présent sur la même plaque.

Le pourcentage obtenu a été utilisé comme mode d'expression des résultats du dosage des anticorps humains spécifiques de la molécule 50 kDa excrétée par Plasmodium falciparum dans le milieu de culture.

2. Résultats

## TABLEAU III

| Groupe | Nombre | ELISA moyenne % référ. | dév.stand. % référ. | Immuno-précipitation 50 & 126 kDa |
|---|---|---|---|---|
| Témoins | 50 | 0,21 | 0,91 | 0/5 |
| Accès palustre (primo-invasion) | 20 | 0,40 | 1,18 | non fait |
| G — 3 à 30 mois | 26 | 2,15 | 3,5 | 5/12 |
| A — 3 à 9 ans | 9 | 17,16 | 11,4 | 7/8 |
| B — Plus de 10 ans | 35 | 36,94 | 33,95 | 7/7 |
| O N — Mères (à l'accouchement) | 19 | 19,42 | 5,46 | 3/3 |
| Cordons (nouveaux-nés) | 19 | 22,9 | 15,83 | 3/3 |

Les résultats résumés dans le tableau III montrent l'intérêt du dosage des anticorps anti 50 kDa de Plasmodium falciparum pour la détermination d'une immunité vis-à-vis de ce parasite.

Le groupe des sujets témoins apparaît négatif. Il en est de même pour le groupe des 20 sujets ayant un accès palustre, si ce n'est le seul malade ayant une primoinfection datant de plusieurs jours pour lequel

13

des anticorps spécifiques ont été trouvés.

Par ailleurs, cette étude montre la présence d'anticorps anti 50 kDa dans le sang de cordon des nouveaux-nés, à un taux comparable à celui des mères au moment de l'accouchement.

L'évolution des anticorps spécifiques de la molécule 50 kDa, mesurés par ELISA, en fonction de l'âge est comparable à l'évolution de l'immunité palustre dans cette région du Gabon : apparition d'une immunité à partir de 3 ans. Cette corrélation a été retrouvée dans une étude récente portant sur 73 enfants originaires de zones holo-endémiques du Nigéria.

V - DOSAGE DE LA MOLECULE 50 KDa EXCRETEE PAR PLASMODIUM FALCIPARUM IN-VITRO DANS LE MILIEU DE CULTURE PAR UNE TECHNIQUE IMMUNO-ENZYMATIQUE (ELISA)

1. Matériel

a) L'anticorps monoclonal.

Le clone 23D5 2H6 a été retenu pour le dosage de l'antigène 50 kDa excrété par Plasmodium falciparum.

b) L'antigène de référence.

L'antigène servant de référence au dosage de la molécule 50 kDa par immunocapture était un surnageant de culture asynchrone de Plasmodium falciparum souche FCR-3. Les conditions de culture ont été les suivantes :

Temps 0 :

hématocrite 6 % (hématies humaines)

parasitémie 7 % (souche FCR-3)

milieu neuf

Récolte à la 24e heure

La culture a été alors centrifugée (600 g pendant 30 minutes à +4°C). Le surnageant ainsi recueilli fut réparti en fractions aliquotes et congelé à -80°C jusqu'au moment de l'utilisation.

Par définition arbitraire, il a été attribué à ce milieu une concentration de 1 unité 50 kDa par ml (1 U50).

c) Le sérum polyclonal humain.

La détection de l'antigène immunoabsorbé par l'anticorps monoclonal a été réalisée grâce au sérum hyperimmun humain servant de référence dans cette étude, décrit plus haut.

d) La technique immunoenzymatique.

La quantification de l'antigène 50 kDa de Plasmodium falciparum a été réalisée en plaques de microtitration à fond plat (MICRO ELISA NUNC Poly labo Block).

Ces plaques ont été sensibilisées sous un volume de 200 $\mu$l, sans prétraitement préalable, par une solution d'IgGs purifiées de l'hybridome 23D5 2H6 (10 gamma/ml) en tampon PBS 0,15 M, pH 9,6). Après une incubation à +4°C, supérieure à 18 heures, les cupules ont été lavées en tampon PBS, pH 7,2 0,15 M (PBS), puis saturées pendant 2 heures à 37°C par une solution à 5 % d'albumine bovine en PBS (200 $\mu$l par cupule).

Les plaques ont alors été lavées avec une solution à 0,05 % de Tween 20 (SERVA) en eau déminéralisée, puis séchées rapidement par retournement (lavage).

Une gamme de dilutions en PBS contenant 0,1 % de Tween 20 (SERVA) et 2 % d'albumine bovine (PBS T A) a été réalisée tant à partir de l'antigène de référence (dilutions 1/10 à 1/1280) qu'à partir des différents milieux testés. Chaque dilution a été testée dans deux cupules différentes (duplicat) sous un volume de 200 $\mu$l. Les plaques ainsi remplies ont alors été mises à incuber pendant une nuit à +4°C.

Après cette incubation, les plaques ont été lavées. 200 $\mu$l d'une dilution en PBS-T-A du sérum de référence ont été versés dans chaque cupule testée. Les plaques ont alors été laissées à +37°C pendant 2 heures.

Un lavage identique a précédé le dépôt de l'antiglobuline conjuguée à la peroxydase (anticorps de chèvre anti chaîne gamma humaine), (TAGO, BIOSOFT, Paris) : 200 $\mu$l par cupule, dilution 1/3000 en PBS-T-A.

Après une incubation de 90 minutes à 37°C, les plaques ont été lavées. La présence de la peroxydase a été révélée par oxydation de l'ortho-phénylène diamine (OPD) en versant dans chaque cupule 200 $\mu$l de la solution suivante, préparée extemporanément :

tampon citrate-phosphate pH 5 ........ 10 ml

ortho-phénylène diamine ............. 4 mg

eau oxygénée 110V ................... 15 $\mu$l

Après une incubation de 10 minutes à température ambiante et à l'obscurité, la réaction enzymatique a été arrêtée par un apport de 50 $\mu$l d'acide sulfurique 2N dans chaque cupule.

La densité optique de chaque cupule a été mesurée directement à travers la plaque par un photomètre (DYNALAB) à la longueur d'onde de 492 nm.

e) Quantification de la concentration de la molécule 50 kDa de Plasmodium falciparum.

Pour chaque dilution testée, la moyenne des densités optiques (DO) du duplicat correspondant a été calculée et diminuée de la densité optique du milieu témoin négatif.

A partir des différentes dilutions du milieu de référence, une courbe étalon établissant une relation entre la DO et le logarithme de la concentration en molécule 50 kDa de ce milieu a été établie (programme sur ordinateur Apple IIe). Seules ont été considérées les concentrations en molécule 50 kDa correspondant à la partie linéaire de courbe.

Pour les milieux étudiés, une courbe analogue a été construite. Seuls les points correspondants à la portion linéaire de la courbe DO / logarithme de la dilution du milieu ont été considérés dans le calcul de la concentration en molécule 50 kDa de Plasmodium falciparum, par comparaison avec la courbe étalon précédemment décrite.

La concentration mesurée la moyenne des concentrations mesurées à partir de ces différents points.


2) Résultats:


Dans cet exemple, la gamme étalon a été établie à partir de dilutions du milieu de référence comprises entre 1/10 et 1/640. L'analyse statistique montre qu'il existe une relation linéaire ($P<0,1$) entre la DO (à 492 nm) et le logarithme de la dilution dans cette gamme, relation qui est confirmée lorsque que l'on porte sur un graphique les variations de la densité optique en fonction du logarithme de la dilution.

Une culture de Plasmodium falciparum a été suivie pendant quatre jours. Le milieu a été entièrement changé à des temps précis tandis que la parasitémie était mesurée. Les surnageants ont été congelés à -80 °C et analysés simultanément à partir de 4 dilutions (1/10 à 1/80). Le tableau IV rapporte en fonction du temps de culture et de la parasitémie de départ (hématocrite constant à 6%), la concentration en molécule 50 kDa libérée par la souche FCR-3 de Plasmodium falciparum dans le surnageant de culture in vitro.

L'étude des résultats ainsi obtenus a permis de montrer la très bonne reproductibilité de la méthode, sa sensibilité et la relation entre le temps de culture, la parasitémie et la quantité de molécule 50 kDa libérée dans le milieu de culture.

L'unité 50 kDa (U50) a pu ainsi être définie comme la quantité de molécule 50 kDa libérée en 24 heures par une culture de la souche FCR-3 de Plasmodium falciparum dans un volume de 1 ml, à 6% d'hématocrite et à 7%de parasitémie.

Il est entendu que la molécule 50 kDa est ici considérée en tant que telle ou comme faisant partie de son précurseur de 126 kDa, dans la mesure où ce dernier est présent à l'état de traces dans les surnageants de culture et où l'anticorps monoclonal reconnaît indifféremment les deux molécules.

TABLEAU IV

**Etude de la libération de la molécule 50 kDa dans le sur-nageant d'un milieu de culture en fonction du temps et de la parasitémie.**

| Exp. | Durée de culture (h) | Parasitémie initiale % | Concentration U 50 kDa / ml |
|------|------|------|------|
| 1 | 24 | O | O |
| 2 | 16,5 | O,11 | O,03 |
| 3 | 7,5 | O,21 | O,03 |
| 4 | 7,5 | O,44 | O,07 |
| 5 | 6,5 | O,75 | O,05 |
| 6 | 16,5 | 1,68 | O,25 |
| 7 | 7,2 | 2,75 | O,13 |
| 8 | 17,7 | 5,O5 | O,55 |

**Ce tableau montre que la concentration en molécule 50 kDa augmente d'une part avec la durée de culture et d'autre part avec la parasitémie initiale.**

Ce tableau montre que la concentration en molécule 50 kDa augmente d'une part avec la durée de culture et d'autre part avec la parasitémie initiale.

VI DOSAGE DE LA MOLECULE 47 kDa EXCRETEE PAR PLASMODIUM FALCIPARUM IN VITRO DANS LE MILIEU DE CULTURE

Ce dosage est effectué selon le protocole décrit sous V mais en utilisant un anticorps monoclonal spécifique de la molécule 47 kDa, par exemple le clone 3E9/1 A11 décrit dans le tableau I.

VII ETUDE DE L'EVOLUTION DU PALUDIME CHEZ UN MALADE PAR DOSAGE DE LA CONCENTRATION EN MOLECULE 50 kDa (OU 47 kDa) DANS LE SERUM

L'étude effectuée a porté sur trois malades au cours du traitement par des antipaludéens classiques (sulfate de quinine et chloroquine). Le dosage sur le sérum est effectué dans les mêmes conditions que décrit plus haut (V ou VI) dans le cas du milieu de culture, mais à des dilutions du sérum de 1/40 et 1/10.

On a observé que pendant la phase aiguë de la maladie, le sérum des malades contenait 0,1 à 0,3 unité de 50 kDa (U 50) (telle que définie plus haut au paragraphe V) par ml.

En effectuant des dosages réguliers et répétés, on a pu constater que la molécule 50 kDa disparaît progressivement du sérum des malades et que la disparition est totale au bout de 4 à 6 jours de traitement.

VIII ETUDE DE LA MOLECULE 126 kDa ET DE SA RELATION AVEC LA MOLECULE 50 kDa

1. Modalités de la transformation 126 - 50 kDa

a) Moment d'apparition de la molécule 50 kDa

Une culture asynchrone est marquée 6 heures par la méthionine [35]S. Les schizontes sont ensuite isolés par centrifugation sur coussin de métrizamide. Ces schizontes sont alors réincubés pendant 16 heures dans les conditions normales de culture, soit seuls (dans ce cas, seule la libération des mérozoïtes intervient) soit en présence d'hématies saines (dans ce cas, la réinvasion se produit). En fin d'incubation, le milieu de culture est immunoprécipité par des IgGs d'un individu immun ou par des anticorps monoclonaux provenant d'un clone sélectionné comme précédemment indiqué. Aucune différence n'est observée entre les deux types d'incubation des schizontes : la transformation 125 - 50 kDa est donc une conséquence de la libération des mérozoïtes et non de la réinvasion des érythrocytes.

Au cours de la même expérience, une partie des schizontes radiomarqués est congelée dans le milieu de culture puis incubée 16 heures à 37°C : la molécule 50 kDa n'apparaît pas dans ces conditions alors que la 126 kDa a été libérée dans le milieu lors de la congélation. Il semble donc que la transformation 126 - 50 kDa requière la libération spontanée des mérozoïtes et ne soit pas une simple protéolyse de la 126 kDa dans le milieu de culture.

2. Cinétique de biosynthèse de la molécule 126 kDa

Une culture asynchrone est marquée par la méthionine $^{35}$S pendant 30 minutes, resuspendue dans le milieu de culture et distribuée en 8 volumes égaux qui sont incubés à 37°C et prélevés successivement après 0, 5, 6, 7, 8, 9, 10 et 11 heures de "chasse". Après solubilisation, les cellules et les milieux de chaque prélèvement sont immunoprécipités par un anticorps monoclonal obtenu comme indiqué précédemment.

La protéine 126 kDa apparaît dans les hématies des la fin du marquage et y persiste jusqu'à 10 heures de "chasse". La 50 kDa apparaît dans le milieu à partir de 6 heures de "chasse".

La transformation 126 - 50 kDa s'opérant lors de la phase de libération des mérozoïtes - réinvasion, l'étude cinétique montre que la protéine 126 kDa est synthétisée pendant environ 4 heures, de 32 à 36 heures, le cycle intraérythrocytaire ayant une durée de 42 heures. Cette phase correspond à la phase de multiplication nucléaire au cours de la schizogonie.

3. Localisation de la molécule 126 kDa

a) Hémolyse sélective par la saponine

Une incubation courte (5 minutes, 20°C) dans une solution de saponine à 0,1% lyse les hématies mais n'altère pas les parasites et, en particulier, ne libère pas l'antigène majeur de 195 kDa présent à la surface des schizontes (référence 17).

Des hématies parasitées radiomarquées par la méthionine $^{35}$S (culture asynchrone, marquage 3 heures, "chasse" 2 heures) sont traitées par la saponine 0,1% en PBS à 20°C pendant 5 minutes. Parasites et surnageant de lyse sont alors séparés par centrifugation et analysés par immunoprécipitation par un anticorps monoclonal obtenu comme indiqué plus haut.

La molécule 126 kDa est retrouvée dans le surnageant saponine, ce qui suggère une localisation vacuolaire (ou dans la membrane de la vacuole parasitophore).

b) Microscopie électronique

Cette technique permet, en utilisant ici encore un anticorps monoclonal, de localiser la molécule 126 kDa à la périphérie de schizontes, au niveau de la vacuole parasitophore, (détection par "immunoperoxydase indirecte" après fixation au paraformaldéhyde-glutaraldéhyde et perméabilisation à la saponine).

IX - PRODUCTION D'ANTICORPS POLYCLONAUX SPECIFIQUES DE LA PROTEINE DE 126 kDa ET IDENTIFICATION DANS LE MILIEU DE CULTURE, D'UN PRODUIT DE 18 kDa EN PROVENANT.

Environ un mg de la protéine 126 kDa purifiée comme précisé au chapître III PURIFICATION DES ANTIGENES 50, 47 et 126 kDa sont injectés à un lapin selon la méthode décrite par VAITUKAITIS (réf. 20), les anticorps recueillis sont utilisés pour immunoprécipiter le surnageant d'une culture marquée par la métionine comme cela a été décrit au chapître VIII paragraphe 1 Moment d'apparition de la molécule 50 kDa. Les résultats de l'immunoprécipitation montrent que le sérum polyclonal préparé chez le lapin contre la molécule de 126 kDa reconnaît en conditions réductrices, trois polypeptides de masses moléculaires respectives 50 kDa, 47 kDa et 18 kDa. Ceci démontre que la transformation de la molécule de 126 kDa donne naissance à trois fragments respectivement 50, 47 et 18 kDa. Une précision supplémentaire est apportée par l'électrophorèse en conditions non réductrices, où l'anticorps polyclonal reconnait une molécule de 50 kDa et une molécule d'environ 65 kDa qui est donc constituée par la réunion des molécules 47 et 18 kDa par un pont disulfure.

Références bibliographiques

(1) J.B. Jensen et W. Trager, American Journal of Tropical Medicine and Hygien (1978), 27 (4), 743-6
(2) G.E. Moore et coll. JAMA, 199, 519(1967)
(3) W. Trager et J.B. Jensen, Science 193, 673-5(1976)
(4) A. Vernes et coll., Am. J. Trop. Med. Hyg. 33, 197-203 (1984).
(5) A.H. Erickson et G. Blobel, J. Biol. Chem. 254, 11771-4 (1979)
(6) J. Saint Blancard et coll., Affinity Chromatography and Related Techniques, Elsevier, Amsterdam (1982) p. 305-312
(7) C.D. Deaton et coll., Clin. Chem. 22, 1465-71(1976)
(8) U.K. Laemmli, Nature, 227, 680-5 (1970)
(9) R.A. Laskey et coll., Eur. J. Biochem. 56, 335-341 (1975)
(10) J.A. Garrels, J. Biol. Chem. 254, 7961-7977 (1979)
(11) P.H. O'Farrel, J. Biol. Chem. 250, 4007-4021 (1975)
(12) G. Kohler et coll., European J. of Immunology, 6, 292, (1976)
(13) Galfre et coll., Nature, 226, 550 (1977)
(14) Fazekas de St. Groth et coll., J. of Immunological Methods, 35, 1 (1980)
(15) R. Dulbecco et coll., Virology 8, 396 (1959)
(16) J. Littlefield, Science 145, 709 (1964).
(17) R.J. Howard et coll., Molec. Bioch. Parasit. 11, 349-362 (1984).
(18) Rodriguez Da Silva et coll., Bull WHO, 61, 105-112 (1983).
(19) ITUX-1 souche isolée à Manaos (Brésil) et communiquée par le Walter Keed Army Institute of Research, Washington U.S.A.
(20) J. Vaitukaitis et coll., J. of Clin. Endocrinol. Metabol. 33, 988-991 (1971)

**Revendications**

**1.** Antigènes pouvant être isolés à partir de la phase intraérythrocytaire de Plasmodium falciparum, caractérisés en ce qu'ils consistent en :
a) une protéine de 126 kDa synthétisée pendant la phase de multiplication nucléaire au cours de la schizogonie et localisée à la périphérie des schizontes, au niveau de la vacuole parasitophore ;
b) une protéine de 50 kDa présentant un point isoélectrique de 5,5, apparaissant dans le sérum des malades infectés par Plasmodium falciparum ou dans le milieu de culture de ce microorganisme, au cours de sa phase intraérythrocytaire et ayant pour précurseur la protéine de 126 kDa définie sous a) ; et
c) une protéine de 47 kDa se résolvant, par électrophorèse bidimensionnelle en deux isophormes de points isoélectriques respectifs 5,9 et 6,1 environ, apparaissent dans le sérum des malades infectés par Plasmodium falciparum ou dans le milieu de culture de ce microorganisme, au cours de sa phase intraérythrocytaire et ayant pour précurseur la protéine de 126 kDa définie sous a).

**2.** Antigène pouvant être isolé à partir de la phase intraérythrocytaire de Plasmodium falciparum, caractérisé en ce qu'il consiste en une protéine de 126 kDa synthétisée pendant la phase de multiplication nucléaire au cours de la schizogonie et localisée à la périphérie des schizontes, au niveau de la vacuole parasitophore et qui est le précurseur d'une protéine de 50 kDa présentant un point isoélectrique de 5,5 et d'une protéine de 47 kDa se résolvant, par électrophorèse, en deux taches à 5,9 et 6,1 environ, lesquelles protéines apparaissent dans le milieu de culture de Plasmodium falciparum et dans le sérum des malades infectés par ce microorganisme.

**3.** Antigène pouvant être isolé à partir de la phase intraérythrocytaire de plasmodium falciparum, caractérisé en ce qu'il consiste en une protéine de 50 kDa présentant un point isoélectrique de 5,5 apparaissant dans le sérum des malades infectés par Plasmodium falciparum ou dans le milieu de culture de ce microorganisme, au cours de sa phase intraérythrocytaire.

**4.** Antigène intraérythrocytaire de Plasmodium falciparum, caractérisé en ce qu'il consiste en une protéine de 47 kDa se résolvant, par électrophorèse bidimensionnelle en deux isophormes de points isoélectriques respectifs 5,9 et 6,1 environ, apparaissant dans le sérum des malades infectés par Plasmodium falciparum ou dans le milieu de culture de ce microorganisme, au cours de sa phase intraérythrocytaire.

5. Antigène selon la revendication 1, caractérisé en ce qu'il consiste également en

d) une protéine de 18 kDa, apparaissant dans le sérum des malades infectés par Plasmodium falciparum ou dans le milieu de culture de ce microorganisme, au cours de sa phase intraérythrocytaire et ayant pour précurseur la protéine définie dans a).

6. Antigène intraérythrocytaire de Plasmodium falciparum caractérisé en ce qu'il consiste en une protéine de 18 kDa apparaisant dans le sérum des malades infectés par Plasmodium falciparum ou dans le milieu de culture de ce microorganisme au cours de sa phase intraérythrocytaire.

7. Antigène de Plasmodium falciparum, caractérisé en ce qu'il consiste en une protéine d'environ 65 kDa formée par la réunion de la protéine de 47 kDa définie sous c) et de la protéine de 18 kDa définie sous d), par des ponts disulfure.

8. Antigènes dérivés des antigènes selon l'une quelconque des revendications 1 à 7, caractérisés en ce qu'ils sont reconnus par un sérum polyclonal obtenu par immunisation par la protéine de 126 kDa.

9. Procédé pour la purification de l'antigène selon l'une des revendications 3, 4 et 6 à 8, caractérisé en ce qu'il consiste essentiellement à faire passer le surnageant d'un milieu de culture asynchrone de Plasmodium falciparum sur une colonne de chromatrographie d'affinité couplée avec des anticorps monoclonaux et/ou polyclonaux spécifiques de la protéine à purifier, et à séparer ensuite l'antigène de la colonne.

10. Procédé pour la purification de l'antigène selon l'une des revendications 2, 5 et 8, caractérisé en ce qu'il consiste essentiellement à faire passer la solution obtenue par solubilisation d'hématies parasitées par Plasmodium falciparum sur une colonne de chromatographie d'affinité couplée avec des anticorps monoclonaux et/ou polyclonaux spécifiques de l'un et/ou l'autre des antigènes 50 kDa, 65, 47 et 18 kDa dont la protéine de 126 kDa est le précurseur, et à séparer ensuite l'antigène de la colonne.

11. Procédé pour le dosage des anticorps contre l'une au moins des protéines selon l'une des revendications 1 à 8, caractérisé en ce qu'il comprend essentiellement les étapes consistant à :

1) fixer sur un support un anticorps monoclonal spécifique de la molécule (47, 18, 65, 50 et/ou 126 kDa) correspondant à l'anticorps à doser ;

2) saturer les sites libres restant sur le support ;

3) faire réagir avec un excès d'antigène obtenu à partir du surnageant d'une culture asynchrone de Plasmodium falciparum ;

4) faire réagir l'antigène fixé sur le support à l'étape 3) avec le milieu à tester ;

5) faire réagir l'ensemble avec un anticorps antiimmunoglobulines humaines, marqué par un enzyme ;

6) déterminer la quantité d'anticorps spécifiques présente dans le milieu étudié, par l'intermédiaire de la quantité d'enzyme fixée sur le support à l'étape 5), au moyen d'une réaction spécifique de l'enzyme, grâce à un étalonnage préalable.

12. Procédé pour le dosage des antigènes selon l'une des revendications 3 à 8 dans le sérum des malades atteints de paludisme ou dans le milieu de culture de Plasmodium falciparum, ou de l'antigène selon la revendication 2, dans les érythrocytes, après solubilisation, caractérisé en ce qu'il comprend essentiellement les étapes consistant à :

1) fixer sur un support un anticorps monoclonal spécifique de la molécule à doser ;

2) saturer les sites libres restant sur le support ;

3) faire réagir avec le milieu susceptible de contenir l'antigène à doser ;

4) faire réagir l'ensemble avec du sérum de référence provenant d'un sujet immun ;

5) faire réagir avec une antiglobuline anti immunoglobulines humaines, marquée par une enzyme ;

6) déterminer la quantité d'antigène présente dans le milieu étudié, par l'intermédiaire de l'activité de l'enzyme, au moyen d'un étalonnage préalable.

13. Vaccin contre le paludisme, caractérisé en ce qu'il comporte, en tant que principe actif, au moins l'un des protéines 18 kDa, 65 kDa, 47 kDa, 50 kDa et 126 kDa définies dans les revendications 1 à 8, de préférence en présence d'un adjuvant, de préférence de l'hydroxyde d'aluminium.

**14.** Dose de vaccin de 0,5 ml, caractérisée en ce qu'elle contient
- de 0,5 à 1,5 mg, de préférence 1 mg, de protéine 18 kDa et/ou 50 kDa et/ou 47 kDa et/ou 65 kDa et/ou 126 kDa définie dans les revendications 1 à 8,
- de 0,3 à 2 mg, de préférence 1 mg, d'hydroxyde d'aluminium et
- une quantité suffisante de soluté isotonique tamponé.

**Claims**

**1.** Antigens that can be isolated from the intraerythrocytic phase of Plasmodium falciparum, characterized in that they consist of :
a) a protein of 126 kDa synthesized during the nuclear multiplication phase during schizogony and localized on the periphery of the schizonts at the parasitophore vacuole level ;
b) a protein of 50 kDa having and isoelectric point of 5.5 appearing in the serum of patients infected by Plasmodium falciparum or in the culture medium of this microorganism during its intraerythrocytic phase and having as precursor the protein of 126 kDa defined under a) ; and
c) a protein of 47 kDa separated by two-dimensional electrophoresis into two isophorms having respectively isoelectric points of about 5.9 and 6.1, appearing in the serum of patients infected by Plasmodium falciparum or in the culture medium of this microorganism during its intraerythrocytic phase and having as precursor the protein of 126 kDa defined under a).

**2.** An antigen that can be isolated from the intraerythrocytic phase of Plasmodium falciparum, characterized in that it consists of a protein of 126 kDa synthesized during the nuclear multiplication phase during schizogony and localized on the periphery of the schizonts, at the parasitophore vacuole level and which is the precursor of a protein of 50 kDa with an isoelectric point of 5.5 and a protein of 47 kDa separated by electrophoresis into two spots of about 5.9 and 6.1, which proteins appear in the culture medium of Plasmodium falciparum and in the serum of patients infected by this microorganism.

**3.** An antigen that can be isolated from the intraerythrocytic phase of Plasmodium falciparum, characterized in that it consists of a protein of 50 kDa with an isoelectric point of 5.5, appearing in the serum of patients infected by Plasmodium falciparum or in the culture medium of this microorganism during its erythrocytic phase.

**4.** An intraerythrocytic antigen of Plasmodium falciparum, characterized in that it consists of a protein of 47 kDa separated by two-dimensional electrophoresis into two isophorms with respective isolectric points of about 5.9 and 6.1, appearing in the serum of patients infected by Plasmodium falciparum or in the culture medium of this microorganism during its intraerythrocytic phase.

**5.** An antigen according to claim 1, characterized in that it also consists of,
d) a protein of 18 kDa, appearing in the serum of patients infected by Plasmodium falciparum or in the culture medium of this microorganism during its intraerythrocytic phase and having as its precursor the protein defined under a).

**6.** An intraerythrocytic antigen of Plasmodium falciparum, characterized in that it consists of a protein of 18 kDa, appearing in the serum of patients infected by Plasmodium falciparum or in the culture medium of this microorganism during its intraerytrocytic phase.

**7.** An antigen of Plasmodium falciparum, characterized in that it consists of a protein of about 65 kDa formed by the combination of the protein of 47 kDa defined under c) with the protein of 18 kDa defined under d) by means of disulfide bonds.

**8.** Antigens derived from the antigens according to any one of the claims 1 to 7, characterized in that they are recognized by a polyclonal serum obtained by immunization by the protein of 126 kDa.

**9.** A method for the purification of the antigen according to any one of the claims 3, 4 and 6 to 8, characterized in that it comprises essentially passing the supernatant of an asynchronous culture medium of Plasmodium falciparum through an affinity chromatography column linked with specific monoclonal and/or polyclonal antibodies of the protein to be purified and then separating the antigen from the column.

**10.** A method for the purification of the antigen according to any one of the claims 2, 5 and 8, characterized in that it comprises essentially passing the solution obtained by the solubilisation of red cells parasitized by Plasmodium falciparum through an affinity chromatography column linked with specific monoclonal and/or polyclonal antibodies of one and/or other of the 50, 65, 47 and 18 kDa antigens of which the protein of 126 kDa is the precursor, and then separating the antigen from the column.

**11.** A method for the assay of antibodies against at least one of the proteins according to any one of the claims 1 to 8, characterized in that it comprises essentially the following stages :

1) fixing on a support a specific monoclonal antibody of the product (47, 18, 65, 50 and/or 126 kDa) corresponding to the antibody to be assayed ;

2) saturating the remaining free sites on the support;

3) reacting with an excess of antigen obtained from the supernatant of an asynchronous culture of Plasmodium falciparum ;

4) reacting the antigen fixed on the support at stage 3) with the medium to be tested ;

5) reacting the whole with an enzyme-labelled human anti-immunoglobulins antibody ;

6) determining the quantity of specific antibodies present in the mediums studied from the quantity of enzyme fixed on the support at stage 5), by means of a specific reaction of the enzyme, thanks to prior standardization.

**12.** A method for the assay of the antigens according to the claims 3 to 8 in the serum of patients suffering from malaria or in the culture medium of Plasmodium falciparum, or of the antigen according to claim 2, in the erythrocytes, after solubilisation, characterized in that it comprises essentially the following stages :

1) fixing on a support a specific monoclonal antibody of the product to be assayed ;

2) saturating the remaining free sites on the support;

3) reacting with the medium likely to contain the antigen to be assayed ;

4) reacting the whole with the reference serum derived from an immune subject ;

5) reacting with an enzyme-labelled anti-human IgGs antiglobulin ;

6) determining the quantity of antigen present in the medium studied through the activity of the enzyme by means of prior standardization.

**13.** A vaccine against malaria, characterized in that it comprises as active principle at least one of the 18 kDa, 65 kDa, 47 kDa, 50 kDa and 126 kDa proteins defined in claims 1 to 8, preferably in the presence of an adjuvant, preferably aluminium hydroxide.

**14.** A 0.5 ml dose of vaccine, characterized in that it comprises :

- 0.5 to 1.5 mg, preferably 1 mg, of 18 kDa and/or 50 kDa and/or 47 kDa and/or 65 kDa and/or 126 kDa protein defined in claims 1 to 8 ;
- 0.3 to 2 mg, preferably 1 mg, of aluminium hydroxide and
- a sufficient quantity of buffered isotonic aqueous solution.

**Patentansprüche**

**1.** Antigene, die von der intraerythrozytären Phase von Plasmodium falciparum isoliert werden können, dadurch gekennzeichnet, daß sie bestehen aus:

(a) einem Protein (126 kDa), das während der Kernvervielfältigungsphase im Verlauf der Schizogonie gebildet wird und an der Peripherie der Schizonten im Bereich der parasitophoren Vacuole lokalisiert ist;

(b) einem Protein (50 kDa) mit einem isoelektrischen Punkt von 5,5, das im Serum von Kranken auftritt, die durch Plasmodium falciparum infiziert sind, oder in einem Kulturmedium des Mikroorganismus im Verlauf der intraerythrozytären Phase und das Protein (126 kDa) gemäß (a) als Vorläufer hat; und

(c) einem Protein (47 kDa), das sich durch zweidimensionale Elektrophorese in zwei Isoformen mit isoelektrischen Punkten von etwa 5,9 bzw. etwa 6,1 auftrennen läßt, im Serum von Kranken, die durch Plasmodium falciparum infiziert sind, oder im Kulturmedium des Mikroorganismus im Verlauf seiner intraerythrozytären Phase auftritt und das Protein (126 kDa) gemäß (a) als Vorläufer hat.

**2.** Antigen, das von der intraerythrozytären Phase von Plasmodium falciparum isoliert werden kann, dadurch gekennzeichnet, daß es

- aus einem Protein (126 kDa), das während der Kernvervielfältigungsphase im Verlauf der Schizogonie gebildet wird und an der Peripherie der Schizonten im Bereich der parasitophoren Vacuole vorliegt und bei dem es sich um einen Vorläufer eines Proteins (50 kDa) mit einem isoelektrischen Punkt von 3,3 handelt und

- aus einem Protein (47 kDa) besteht, das sich durch Elektrophorese in zwei Banden bei etwa 5,9 und etwa 6,1 auftrennen läßt, wobei diese Proteine im Kulturmedium von Plasmodium falciparum und im Serum von Kranken auftreten, die durch den Mikroorganismus infiziert sind.

3. Antigen, das aus der intraerythrozytären Phase von Plasmodium falciparum isoliert werden kann, dadurch gekennzeichnet, daß es aus einem Protein (50 kDa) mit einem isoelektrischen Punkt von 5,5 besteht, das im Serum von Kranken, die durch Plasmodium falciparum infiziert sind, oder im Kulturmedium des Mikroorganismus während der intraerythrozytären Phase auftritt.

4. Intraerythrozytäres Antigen von Plasmodium falciparum, dadurch gekennzeichnet, daß es aus einem Protein (42 kDa) besteht, das sich durch zweidimensionale Elektrophorese in zwei Isoformen mit einem isoelektrischen Punkt von etwa 5,9 bzw. etwa 6,1 auftrennen läßt und im Serum von Kranken, die durch Plasmodium falciparum infiziert sind, oder im Kulturmedium des Mikroorganismus im Verlauf seiner intraerythrozytären Phase auftritt.

5. Antigen nach Anspruch 1, dadurch gekennzeichnet, daß es ferner

(d) aus einem Protein (18 kDa) besteht, das im Serum von Kranken, die durch Plasmodium falciparum infiziert sind, oder im Kulturmedium des Mikroorganismus im Verlauf seiner intraerythrozytären Phase auftritt und als Vorläufer das Protein gemäß (a) hat.

6. Intraerythrozytäres Antigen von Plasmodium falciparum, dadurch gekennzeichnet, das es aus einem Protein (18 kDa) besteht, das im Serum von Kranken, die durch Plasmodium falciparum infiziert sind, oder im Kulturmedium des Mikroorganismus im Verlauf seiner intraerythrozytären Phase auftritt.

7. Antigen von Plasmodium falciparum, dadurch gekennzeichnet, daß es aus einem Protein (etwa 65 kDa) besteht, das durch Vereinigung des Proteins (47 kDa) gemäß (c) mit dem Protein (18 kDa) gemäß (d) über Disulfidbrücken gebildet wird.

8. Antigene, die sich von Antigenen gemäß einem der Ansprüche 1 bis 7 ableiten, dadurch gekennzeichnet, daß sie durch ein polyklonales Serum erkannt werden, das durch Immunisierung mit Hilfe des Proteins (128 kDa) erhalten wird.

9. Verfahren zur Reinigung des Antigens gemäß einem der Ansprüche 3, 4 und 6 bis 8, dadurch gekennzeichnet, daß es im wesentlichen darin besteht, daß man den Überstand eines asynchronen Kulturmediums von Plasmodium falciparum eine Affinitätschromatographiesäule passieren läßt, die monoklonale und/oder polyklonale und für das zu reinigende Protein spezifische Antikörper trägt, und danach das Antigen von der Säule abtrennt.

10. Verfahren zur Reinigung des Antigens gemäß einem der Ansprüche 2, 5 und 8, dadurch gekennzeichnet, daß es im wesentlichen darin besteht, daß man eine Lösung, die durch Solubilisieren von roten Blutkörperchen erhalten worden ist, die durch Plasmodium falciparum parasitiert sind, eine Affinitätschromatographiesäule passieren läßt, die monoklonale und/oder polyklonale Antikörper trägt, die für das eine und/oder das andere der Antigene von 50 kDa, 65 kDa, 47 kDa und 18 kDa spezifisch sind, deren Vorläufer das Protein (126 kDa) ist, und danach das Antigen von der Säule abtrennt.

11. Verfahren zur Quantifizierung oder Dosierung von Antikörpern gegen mindestens eines der Proteine gemäß einem der Ansprüche 1 bis 8, dadurch gekennzeichnet, daß es im wesentlichen die folgenden Schritte umfaßt:

1) Fixieren eines monoklonalen Antikörpers, der für das Molekül von 47, 18, 65, 50 und/oder 126 kDa in Übereinstimmung mit dem zu dosierendem Antikörper spezifisch ist, auf einem Träger;

2) Sättigen der freien Stellen, die auf dem Träger verblieben sind,

3) Umsetzen mit einem Überschuß des Antigens, das aus dem Überstand einer asynchronen Kultur von Plasmodium falciparum erhalten worden ist;

4) Umsetzen des Antigens, das auf dem Träger in der Stufe (3) fixiert worden ist mit dem zu testenden Medium,

5) Umsetzen des Systems mit einem Human-Antiimmunoglobulin-Antikörper (markiert durch ein Enzym),

6) Bestimmen der Menge der spezifischen Antikörper, die in dem zu untersuchenden Milieu vorliegen, mit Hilfe der Menge des Enzyms, das auf dem Träger in der Stufe (5) fixiert worden ist, mit Hilfe einer Reaktion, die für das Enzym spezifisch ist (nach vorhergehender Eichung).

12. Verfahren zur Quantifizierung oder Dosierung von Antigenen gemäß einem der Ansprüche 3 bis 8 im Serum von Kranken, die von Malaria befallen sind oder im Kulturmedium von Plasmodium falciparum, oder eines Antigens gemäß Anspruch 2 in Erythrozyten nach Solubilisierung, dadurch gekennzeichnet, daß das Verfahren im wesentlichen aus den folgenden Schritten besteht,

(1) Fixieren eines monoklonalen Antikörpers, der für das zu dosierende Molekül spezifisch ist, auf einen Träger

(2) Sättigen der freien Stellen, die auf dem Träger verblieben sind

(3) Umsetzen mit dem Medium, das das zu dosierende Antigen enthalten kann

(4) Umsetzen des Systems mit dem Bezugsserum, das in einem immunen Subjekt vorkommen kann

(5) Umsetzen mit einem Human-Antiimmunoglobulin-Antiglobulin, das durch ein Enzym markiert ist

(6) Bestimmen der antigenen Menge im zu untersuchenden Medium mit Hilfe der Aktivität des Enzyms (mit vorhergehender Eichung).

13. Impfstoff gegen Malaria, dadurch gekennzeichnet, daß er als Wirkstoff mindestens ein Protein von 18 kDa, 65 kDa, 47 kDa, 50 kDa und 126 kDa gemäß einem der Ansprüche 1 bis 8 vorzugsweise in Gegenwart eines Hilfsmittels und vorzugsweise in Gegenwart von Aluminiumhydroxid umfaßt.

14. Impfstoffdosis von 0,5 ml, dadurch gekennzeichnet, daß sie folgende Komponenten enthält:
- 0,5 bis 1,5 mg und vorzugsweise 1 mg Protein von 18 kDa und/oder 50 kDa und/oder 47 kDa und/oder 65 kDa und/oder 126 kDa gemäß einem der Ansprüche 1 bis 8,
- 0,3 bis 2 mg und vorzugsweise 1 mg Aluminiumhydroxid und
- eine ausreichende Menge einer isotonischen Pufferlösung